# EUROPÄISCHE PATENTSCHRIFT

(11) **EP 0 732 936 B1**
(45) Veröffentlichungstag und Bekanntmachung des Hinweises auf die Patenterteilung: **29.03.2000**
(21) Anmeldenummer: 95903236.8
(22) Anmeldetag: 07.12.1994
(51) Int. Cl.: A61K 39/39

(54) **ADJUVANS FÜR ANTIGENE, VERFAHREN ZUR HERSTELLUNG UND VERWENDUNG**
ADJUVANT FOR ANTIGENS, PROCESS FOR PRODUCING THE SAME AND ITS USE
ADJUVANT POUR ANTIGENES, SON PROCEDE DE PRODUCTION ET SON UTILISATION

(30) Priorität: 09.12.1993 DE 4341938; 05.12.1994 DE 4445074
(43) Veröffentlichungstag der Anmeldung: 25.09.1996
(73) Patentinhaber: Exner, Heinrich, 39291 Möckern (DE)
(72) Erfinder: Exner, Heinrich, 39291 Möckern (DE)
(74) Vertreter: Fleischer, Harald
(86) Internationale Anmeldenummer: DE9401495
(87) Internationale Veröffentlichungsnummer: WO9515768

(56) Entgegenhaltungen:
- EP-A- 0 013 851
- DD-A- 265 992
- FR-A- 2 446 111
- US-A- 3 577 524

## Beschreibung

Die Erfindung bezieht sich auf Adjuvantien für Antigene wie Viren, Bakterien, Parasiten einschließlich ihrer Stoffwechselprodukte bzw. Teile der Strukturen von Viren, Bakterien und Parasiten zur Immunisierung, Verfahren zur Herstellung und Verwendungen.

Die Applikation von Impfstoffen erfolgt zur Verbesserung der Antikörperausbildung mit Hilfe von Adjuvantien. Adjuvantien sollen dabei die Resorption parenteral verabreichter Antigene verlangsamen und damit einen langen Antigenreiz ausüben. Dadurch wirken sie als Verstärker immunogener Wirkung von Antigenen.

Bekannt ist das sogenannte Freund-Adjuvans, daß in seiner inkompletten Form eine Mineralöl-in-Wasser-Emulsion darstellt, während die komplette Form unter Zusatz von inaktivierten Mykobakterien zur inkompletten Form hergestellt wird. Die Adjuvanswirkung inaktivierter Mykobakterien wird einer darin enthaltenden Wachsfraktion zugeschrieben. Das komplette Freund-Adjuvans ruft häufig am Ort der Applikation eine Granulombildung hervor. Daher wird das originäre komplette Freund-Adjuvans nur für tierexperimentielle Zwecke verwendet.

In der Medizin werden als Adjuvantien häufig Aluminium-Verbindungen in Form von Hydroxiden oder Phosphaten angewendet. Diese Aluminiumverbindungen ergeben durch Kopplung mit Impf-Antigenen die sogenannten Adsorbat-Impfstoffe, deren immunpotenzierende Wirkung durch entstehende Impfstoffdepots erklärt werden, aus denen das Antigen verzögert zur Resorption freigesetzt wird. Nachteilig beim Einsatz der festen Aluminiumverbindungen ist ihre Neigung zur Aggregation und zur Sedimentation sowie ihr Rückstandsverhalten.

Ein weiterentwickeltes inkomplettes Freund'sches Adjuvans ist eine stabile Wasser-in-Öl-Emulsion, die bekanntermaßen aus Mineralöl, einfachem Emulgator wie Manitolmonooleat, physiologischer Kochsalzlösung und Tween 80 besteht. Diese Komponenten werden in der Regel mit einem Hochleistungshomogenisator dispergiert und mit spezifischen Impfantigenen zur Vakzine komplettiert.

Allen diesen Vakzinen auf der Basis der genannten Wasser-in-Öl-Emulsion ist gemeinsam, daß die Antigenverteilung nur in der wäßrigen Phase der Emulsion erfolgt und damit keine entsprechend lange Depotwirkung des Antigens erreicht wird. Da die Viskosität von Wasser-in-Öl-Emulsionen relativ hoch ist, lassen sie sich schlecht applizieren, d.h. es müssen Kanülen mit großem Kaliber verwendet werden. Hierzu kommt, daß alle eingesetzten Geräte wegen der Verölung einer intensiven Reinigung bedürfen.

Am Ort der Injektion erfolgt möglicherweise eine Granulombildung, die zufolge hat, daß die Antigene über die Lymphbahnen rasch inkorporiert werden.

Die maximale Haltbarkeitsdauer bei Temperaturen von 37°C wird in der Literatur mit einer Woche angegeben.

Danach kommt es zur Trennung und Abscheidung der Wasservon der Ölphase und damit zur Zerstörung der Emulsion.

Nachteilig ist es ebenfalls, daß zur Herstellung dieser Wasser-in-Öl-Emulsionen Hochleistungshomogenisatoren unbedingt erforderlich sind. Ein weiterer Nachteil ist, daß der Transport und die Aufbewahrung der Emulsion ausschließlich bei Kühlschranktemperaturen erfolgen muß.

In der DD 265 992 ist ein Adjuvans für Antigene zur Immunisierung sowie Verfahren zur Herstellung desselben beschrieben. Die hier vorliegende Öl-in-Wasser-Emulsion weist eine niedrige Viskosiät der Vakzine auf, die keine besonderen Anforderungen an die Applikationstechnik stellt. Die Geräte können ohne besonderen Aufwand gereinigt werden und die Verwendung des hier angegebenen dipolaren aprotonischen Lösungsmittels soll die Verteilung des Antigens in der wäßrigen und in der Öl-Phase garantieren und damit den Immunisierungseffekt erhöhen. Zur Herstellung der Öl-in-Wasser-Emulsion sollen relativ geringe mechanische Energien notwendig sein, so daß einfache Rührapparate genügen sollen, um eine Emulsion der dort beschriebenen Art herzustellen. Der Ölanteil der Emulsion besteht aus Mineralöl und kann ggf. auch Silikonöle enthalten.

Nachteilig an diesem Adjuvans ist jedoch, daß die Verwendung von Mineralölen in diesem Falle Paraffinöle, eine Reihe Probleme bezüglich der Stabilität der Öl-in-Wasser-Emulsion mit sich bringt. So ist die Variation der Viskosität des Mineralöls nur in kleinen Grenzen möglich. Das führt dazu, daß bei der Dispergierung Öltröpfchen mit relativ großer Teilchengröße entstehen. Größere Öltröpfchen bewirken eine Erhöhung der Gesamtviskositiät der Emulsion mit den damit verbundenen Nachteilen und sie beeinflußen auch die Stabilität der Emulsion ungünstig, d.h. ein Aufrahmen des Öles läßt sich nicht vermeiden, insbesondere nach längerem Stehen der Emulsion.

Auch die in dieser Öl-in-wasser-Emulsion vorhandene polydisperse Größenverteilung der Öltröpfchen führt zu instabilen Verhältnissen und Histoinkompatibilitäten.

Nachteilig ist weiterhin, das Rückstandsverhalten der Mineralöle, die im Organismus Unverträglichkeiten herbeiführen und die bei der Anwendung im Tier Probleme bei der Verwertung der Tiere für die menschliche Ernährung schaffen können.

Hinzu kommt, daß Mineralöle bestimmte Antigene angreifen und damit Impfstoffe unwirksam machen können.

Auch der Einsatz der sogenannten dipolar-aprotischen Lösungsmitteln wie Dimethylsulfoxid in Verbindung mit Mineralölen bringt nicht die erhoffte Depotwirkung. Offensichtlich ist der Austausch der Antigene zwischen Wasser- und Öl-Phase im Sinne einer langsamen Nachlieferung von Antigenen aus der Öl-Phase in die wäßrige Phase (Boosterung) in Gegenwart eines dipolaraproischen Lösungsmittels nicht optimal gewährleistet.

Aufgabe der Erfindung ist es, Adjuvantien zur Verfügung zu stellen, die durch die Kombination mit Impfantigenen oder in Kombination mit Peptidoglycanen in einer histokompatiblen Formulierung es möglich machen, daß die Abwehrmechanismen im Körper so weit stimuliert werden, daß erstmalig neben der aktiven Immunprophylaxe auch schwacher Antigene die allgemeine und spezifische Immuntherapie möglich wird. Dabei soll die Adjuvansherstellung den allgemein üblichen Aufwand nicht übersteigen und die Applikationsmöglichkeit des Impfstoffes sichern. In relativ immuninkompetenten Lebensabschnitten soll durch eine Kombination der allgemeinen Immunprophylaxe oder alleinige Anwendung des Adjuvans eine hohe Immunkompetenz erreicht werden. Das Rückstandsverhalten des Adjuvans soll keine Probleme mit sich bringen.

Die Lösung der Aufgabe erfolgt mit Adjuvantien für Antigene, ein Verfahren zur Herstellung und Verwendung der Adjuvantien gemäß der kennzeichnenden Teile der Ansprüche 1, 7 und 14 sowie der Verwendungsansprüche 15 und 16.

Die erfindungsgemäßen Adjuvantien sind vom Typ her Öl-in-Wasser-Emulsionen. Die Öl-Phase besteht aus Polydimethylsiloxanen und die wäßrige Phase im wesentlichen aus biokompatibler Salzlösung. Stabilisiert wird die Öl-Phase in der Wasser-Phase mittels eines Komplexemulgators, der einen HLB-Wert von 9-16 aufweist. Der Komplexemulgator ist eine Kombination aus aliphatischen Alkoholen im wesentlichen mit der Kettenlänge C₁₀ bis C₁₀₀ aus Sorbitol und/oder Glycerolfettsäureestern und aus Polysorbaten. Die biokompatible Salzlösung ist eine Phosphat gepufferte Natriumchloridlösung, die Chelatbildner enthält. Erfindungsgemäß enthält die Kombination zusätzlich Dimethylsulfoxid, das sich entsprechend seinem Löslichkeitsverhalten in beiden Phasen verteilt.

Zweckmäßig ist es, mehrwertige, wasserlösliche Alkohole wie Glycerol zuzusetzen. Insbesondere die Chelatbildner bewirken eine weitere Stabilisierung der Emulsion

Erfindungsgemäß wird durch Zusatz von Peptidoglycanen auf der Basis von speziesspezifischen St.aureus-Stämmen und wasserlöslichen natürlichen und/oder synthetischen Polymeren zum inkompletten Adjuvans ein komplettes Adjuvans hergestellt. Die im kompletten Adjuvans anwesenden wasserlöslichen Polymere üben eine zusätzliche stabilisierende Wirkung auf die Emulsion aus. Sie beeinflussen weiterhin außerordentlich günstig die Freigabe der Antigene am Injektionsort sowohl aus der Öl-aus auch aus der Wasserphase.

Die Polydimethylsiloxane sind durch entsprechende Wahl des Polymerisationsgrades bezüglich ihrer Viskosität in weiten Grenzen variierbar.

Es zeigte sich nun überraschend, daß insbesondere die Kombination zwischen Polydimethylsiloxan als Öl-Phase und durch Verwendung des Komplexemulgators eine feinteilige Emulsion mit enger Größenverteilung der Öltröpfchen herstellbar ist, die bei Anwesenheit von Dimethylsulfoxid eine rasche Verteilung des Impf-Depots am Injektionsort bei Erhaltung der Boostereffekte ermöglicht. Dadurch wird eine hohe Histokompatibilität erreicht, die die Voraussetzung für die Anwendung der Polydimethylsiloxane darstellt.

Das Zusammenwirken von Polydimethylsiloxanen und den Peptidoglycanen auf der Basis der St.aureus-Stämmen führt zu einer bemerkenswerten, bisher nicht bekannten Intensität der unspezifischen Immunantwort vorn T- und B-Zell-Typ.

Gemäß der Erfindung erfolgt die Herstellung des kompletten Adjuvans für Antigene wie Viren, Bakterien, Parasiten einschließlich ihrer Stoffwechselprodukte bzw. Teile der Strukturen von Viren, Bakterien und Parasiten zur Immunisierung dadurch, daß zu dem Glycerol enthaltenden inkompletten Adjuvans eine Kochsalz enthaltene wäßrige Zusammensetzung von Peptidoclycanen auf der Basis von spezifischen St.aureus Stämmen und wasserlöslichen- und natürlichen und/oder synthetischen Polymeren zugefügt werden, wobei zur Herstellung der Peptidoglycane speziesspezifische Stämme von St.aureus ausgewählt werden, die auf 5% Blutagar 24 Stunden bebrütet werden. Die geernteten Staphylokokken werden in 0,5% phosphatgepufferten Phenolkochsalziösung aufgenommen und 48 Stunden bei 37°C inaktiviert. Die inaktivierten Staphylokokken werden separiert gewaschen und in einer 5%igen Lösung eines wasserlöslichen natürlichen und/oder synthetischen Polymeres in Kochsalzlösung aufgenommen und autoklaviert. Die Behandlung im Autoklaven erfolgt als optimale Variante bei 121°C. Die Dosierung der Peptidoglycane liegt zwischen 10² und 10⁹ bezogen auf geerntete Keime je ml fertige Vakzine.

Als erfindungsgemäß eingesetzte wasserlösliche Polymere haben sich insbesondere die partialsynthetischen Celluloseester Polyvinylpyrrolidon und Poly-6-Aminohexansäure bewährt.

Die erfindungsgemäß eingesetzten Polydimethylsiloxane weisen verschiedene Vorteile auf. So sind sie insbesondere gegenüber oxidativen und hydrolytischen Einflüssen beständig, sie sind temperaturbeständig und die Viskositätsabhängigkeit von der Temperatur ist gering. Hinzu kommt, daß sie geruchs- und geschmacklos sind. Da die Viskosität mit steigendem Polymerisationsgrad zunimmt, ist es ohne weiteres möglich a priori die gewünschte Viskosität der Emulsion einzustellen. Hinzu kommt, daß die Polydimethylsiloxane im Gegensatz zu einem Mineralöl eine sehr geringe Temperaturabhängigkeit zeigen. So eignet sich insbesondere ein Polydimethylsiloxan mit linearer Kettenstruktur und einem Viskositätswert von 0,0005 m²/s zur Herstellung sehr feinteiliger und im wesentlichen homodisperser Emulsionen. Die relativ niedrige Oberflächenspannung der Polydimethylsiloxane erlaubt es mit geeigneten Komplexemulgatoren erfindungsgemäß durch spontane Emulgierung die gewünschten Verteilungen der Öl-Phase in der wäßrigen Phase zu erreichen. Die dazu erfindungsgemäß eingesetzten Komplexemulgatoren bestehen aus aliphatischen Alkoholen der Kettenlänge C₁₀ bis C₁₀₀, aus Sorbitol und/oder Glycerolfettsäureester und aus Polysorbaten. Ein optimales Verhältnis der genannten drei Bestandteile beträgt 1:1:1.

Zur Charakterisierung der Wirkung der erfindungsgemäßen Komplexemulgatoren dient der HLB-Wert (HYDROPHILE-LIPOPHILE-BALANCE). Ein günstiger HLB-Wert liegt bei 12. Ein optimal wirkender erfindungsgemäßer Komplexemulgator besteht beispielsweise aus Polyoxyethylen-Sorbitol-Hexaoleat, Polyoxyethylen-(20)-Sorbitanmonooleat und Cetylstearylalkohol. Zur Herstellung des kompletten Adjuvanses werden die Peptidoglycane separat oder zusammen mit dem Antigen zur Immunisierung in dem inkompletten Adjuvans im Temperaturbereich von 20-30°C als wäßrige Lösung zugeführt.

Das erfindungsgemäße Adjuvans kann entsprechend seiner Fertigung wäßrige Antigenlösung aufnehmen.

Die erfindungsgemäßen Adjuvantien dienen der Immunprophylaxe in der Human- und Veterinär-Medizin. Sie werden zur Verbesserung der Immunprophylaxe durch aktive Stimulierung der zellulären und humoralen Immunkompetenz eingesetzt und können durch ihren Wirkungsmechanismus zur allgemeinen und spezifischen Immuntherapie eingesetzt werden. Die Formulierung des Adjuvans bewirkt eine rasche Verteilung des Impf-Depots am Injektionsort bei Erhaltung der Boostereffekte. Dadurch wird eine hohe Histokompatibilität erreicht. Die Adjuvansherstellung übersteigt nicht den allgemein üblichen Aufwand und die Applikationsfähigkeit des Impfstoffes. Durch die Verwendung der Polydimethylsiloxane ist eine sehr gute Temperaturbeständigkeit und Stabilität der Öl-in-Wasser-Emulsion gesichert. Insbesondere wird durch die Kombination von erfindungsgemäßem Adjuvans allein mit Impf-Antigenen oder in Kombination mit Peptidoglycanen in einer histokompatiblen Formulierung erreicht, daß die Abwehrmechanismen im Organismus soweit stimuliert werden, daß erstmalig neben der aktiven Immunprophylaxe schwacher Antigene die allgemeine und spezifische Immuntherapie möglich wird.

Der Wirkungsmechanismus des erfindungsgemäßen Adjuvans sichert auch eine Immunstimulation bei nichtinfektiösen Immundepressionen (z.B. Prophylaxe und Therapie dystrophischer Prozesse, Autoimmunerkrankungen, Osteopathien etc.).

In relativ immuninkompetenten Lebensabschnitten soll durch sinnvolle Kombination mit der allgemeinen Immunprophylaxe oder alleiniger Anwendung des bzw. der erfindungsgemäßen Adjuvantien eine hohe Immunkompetenz erreicht werden (Pädiatrie, Geriatrie).

### Beispiel 1

Zur Herstellung von 1000 g erfindungsgemäßem inkompletten Adjuvans werden 5 g Polydimethylsiloxane, 3,5 g Komplexemulgator mit einem HLB-Wert von 12, 2,5 g Glycerol und 2,1 g Dimethylsulfoxid mit 986,9 g biokompatibler Salzlösung, die 0,2g Ethylendiamintetraacetat, Na₂Ca, enthält, verrührt und unter ständigem Rühren auf 100°C erwärmt und bei 121°C und 0,5 Atmosphären autoklaviert und anschließend durch kräftiges Rühren, beginnend bei 90°C und bei weiterer abfallender Temperatur bis unter 30°C, reemulgiert. Das auf diese Weise hergestellte Adjuvans ist bei Ampullenlagerung mehr als 4 Jahre und 4°C lagerfähig.

### Beispiel 2

Zur Herstellung des kompletten Adjuvans werden die Peptidoglycane dem inkompletten Adjuvans, hergestellt nach Beispiel 1, im Temperaturbereich von 20 bis 30°C als wäßrige Lösung zugeführt. Die Peptidoglycankonzentration pro Impfdosis liegt in Abhängigkeit von der Speziesspezifität bei 0,001-50 µg Eiweißstickstoff. Das Adjuvans kann entsprechend seiner Fertigung wäßrige Antigenlösung zur Vakzinfertigung aufnehmen.

Zur Herstellung der Peptidoglycane werden speziesspezifische Stämme von ST.aureus ausgewählt und auf 5% Blutagar 24 Stunden bebrütet. Die geernteten Staphylokokken werden in 0,5% Phosphat gepufferten Phenolkochsalzlösung aufgenommen und 48 Stunden bei 37°C inaktiviert. Die inaktivierten Staphylokokken werden separiert, gewaschen und in einer 5%-igen Polyl(2-oxo-1-pyrrolidinyl) ethylenkochsalzlösung aufgenommen und autoklaviert (121°C). Die Peptidoglycane werden danach abzentrifugiert und in einer phosphatgepufferten Kochsalzlösung aufgenommen und auf einen Stickstoffgehalt von 0,001-50 µg je ID eingestellt. Bei der Auswahl der Stämme von Staphylokokken bzw. Streptokokken sollten bei den jeweiligen Spezies solche Stämme ausgewählt werden, die aus pathogenen Prozessen stammen und noch nach Möglichkeit Hospitalstämme der jeweiligen Art sind. Entsprechend des Einsatzes erfolgt die Abfüllung des kompletten Adjuvans in Impfstofflaschen.

### Beispiel 3

### Tauben:

### Paramyxovirusinfektion

### Status präsens:

Ein Bestand von 300 Tauben war an PMV-Infektion erkrankt. Innerhalb von 4 Wochen verendeten 120 Tiere. Zum Zeitpunkt der Impfung waren von den 180 verbleibenden Tieren 40 klinisch stark erkrankt und die restlichen 140 Tiere morbid.
1 ml inkomplettes Adjuvans (Beispiel 1) mit PMV-Antigenen 10⁶EID pro ml. In die Vakzination wurden alle Tiere einbezogen. Von den 40 schwer erkrankten starben noch 25%, von den übrigen 3% des Bestandes.

### Ergebnis:

Bereits nach 3 bis 5 Tagen stabilisierte sich der Gesundheitszustand erheblich. Das Sterben ließ nach und selbst Tiere mit extrem verändertem Allgemeinbefinden kehrten zur Norm zurück.

### Epikrise:

3 Wochen vor der Immunisierung mit dem inkompletten Adjuvans wurden die Tauben mit einem Wasser-in-Öl-Adjuvans PM-Impfstoff ohne Effekt immunisiert. Deutlich wurde erstmalig, daß mit dem inkompletten Adjuvans und PMV-Antigen in eine Infektion hineingeimpft werden konnte und das klinische Bild sich schlagartig besserte (3 bis 5 Tage p.a.).

### Beispiel 4

### Tauben

### Salmonellose

### Anamnese:

s.t.m. Infektion in einem Bestand von etwa 50 Tieren über mehrere Jahre. Behandlung mit Antibiotika entsprechend Antibiogramm; bisher keine nachhaltigen Effekte. Auch inaktivierte S.t.m. Vakzinen dämmten das Geschehen nicht ein.

### Vakzination:

Immunisierung des Gesamtbestandes mit inkomplettem Adjuvans (Beispiel 1) pro ml und Tier und 10⁹ inaktivierte S.t.m. Keime pro ml brachten einen optimalen Schutz. Eine Boosterung 4 Wochen nach der Erstvakzination brachte eine Verbesserung der Aufzucht junger Tauben und die Eradikation der S.t.m. Infektion.

In die Immunisierung mit inkomplettem Adjuvans wurden die Nestjungen mit guter Verträglichkeit einbezogen.

### Beispiel 5

### Hühner

### Slowvirusinfektion - Broilerelternvermehrungszucht

### Anamnese:

Seit Mitte der achtziger Jahre wird euorpa- und weltweit das Ansteigen des sogenannten Malabsoptionssyndroms (Slowvirusinfektion) beschrieben (Synonyma: Helikopter disease, Brittle bone disease, Standing syndrom, Femoral head necrosis, Leg problems u.a.)
Damit im Zusammenhang traten opportunistische Infektionen wie Marek'sche Krankheit, Kokzidiose, Staph. Arthritis, Salmonellen u.v.a.m. auf.

Klinisch tritt in diesen Herden im Alter von 2 bis 4 Wochen bereits eine erhebliche Wachstumsdifferenzierung auf (Standing Syndrom).
Bis zur Legereife von etwa 20 bis 24 Wochen kann ca 1/4 des Bestandes verlustig gehen.
Ein Bestand von etwa 7000 Tieren hatte in der 3. Lebenswoche bereits 10 bis 15% der Tiere des Bestandes Untergewichte. Die erreichten Gewichte der zurückgebliebenen Tiere lagen bei 65% Gewicht vom Mittelwert der Gruppe.
Die Tiere wachsen erheblich langsamer, schlechte Befiederung und erhöhte Krankheitsanfälligkeit sind die Folge.
Die auf diese Weise ausgesonderten Tiere können in der Aufzucht bis zu 25% ausmachen. Durch die erhöhte Krankheitsanfälligkeit treten in diesen Herden auch nach Legebeginn viele oft kaum zu beherrschende opportunistische Infektionskrankheiten auf. Durch versuchsgemäße Vakzination einer solchen Herde mit komplettem Adjuvans (Beispiel 2) wurden folgende Beobachtungen gemacht:

Nach der Vakzination erholte sich die Herde. die abgemagerten Tiere konnten bei freier Fütterung innerhalb von 10 bis 14 Tagen einen großen Teil ihres Gewichtes aufholen; auch die Morbidität opportunistischer Krankheiten nahm ab.

Bei vergleichendem Einsatz handelsüblicher Ölemulsionsvakzinen trat dieser Effekt nicht auf.

### Nach diesen Beobachtungen wurden weitere Herden mit kompletten Adjuvans wie folgend immunisiert:

Eine Impfdosis komplettes Adjuvans (1 ml) wurde mit je einer Impfdosis ND, IB und IBD versehen (Serologie ND, IB und IBD entsprach im Titer vergleichbaren Ölemulsionsadjuvantien).
Nach den Wachstumskurven erfolgte die erste Immunisierung unmittelbar vor dem ersten Auftreten des Standing Syndroms im Alter von 3 bis 4 Wochen, die zweite Immunisierung im Alter von 10 bis 12 Wochen und die dritte Immunisierung im Alter von 18 bis 20 Wochen mit komplettem Adjuvans.
Nach diesem Immunisierungsprogramm waren die Wachstumskurven wieder eine Gerade, die Verluste gingen auf das normale Maß zurück. Besonders deutlich waren die Effekte bei MK und Leukose. Ohne Änderungen im MK Impfprogramm gingen die Verluste durch Neoplasien gegen Null zurück.

### Epikrise:

Das Standing Syndrom wurde durch die Vakzination mit kompletten Adjuvans aufgehoben und alle anderen Probleme der opportunistischen Krankheiten wurden auf ein Minimum zurückgedrängt.
In vergleichsweise anderen Herden mit mineralölhaltigen Impfstoffen durchgeführten Vakzinationen wurden keine solchen Effekte erzielt.
Durch die Gesundheitsstabilisierung in der Aufzucht wurde eine erheblich Leistungssteigerung bei den erwachsenen Tieren festgestellt.
In den einzelnen Zuchtstufen wurde nach der Immunisierung ein begrenzter Generationeneffekt feststellbar, der über 4 Wochen anhielt und nicht mit maternalen Antikörpern zu deuten ist, d.h. in der nachfolgenden Generation wurden diese frühzeitigen Wachstumsprobleme des Standing Syndroms nicht gesehen.
Gegenwärtig werden in anderen Linien mit gleichen Symptomen diese Effekte reproduziert (ca 30 000 Tiere).

### Beispiel 6

### Furunkulose

### Status präsens:

Patientin mit multiplen Furunkeln am Stamm nach Entbindung.
Bakteriologische Untersuchung ergab St.aureus mit antibiotischer Mehrfachrestistenz.
Die Lysotypie ergab Stammübereinstimmung mit Hospitalkeimen der Entbindungsstation. Die konventionelle Therapie versagte. Eine Autovakzine mit inaktivierten St.aureus Antigen verhinderte das erneute Exazerbieren für maximal 4 Wochen.

### Vakzination:

In 1 ml komplettes Adjuvans (Beispiel 2) wurden 10⁹ inaktivierte Keime des Hospitalstammes St.aureus verbracht.
Die Immunisierung erfolgte mit 1 ml komplettem Adjuvans. Bereits nach drei Tagen begannen die Furunkel einzutrocknen und nach 10 Tagen war die Furunkulose geheilt.
Als ein weiteres Familienmitglied dieser Patientin an Furunkulose erkrankte wurde mit komplettem Adjuvans und inaktivierten St.aureus Antigen mit gleichem Effekt immunisiert.

### Beispiel 7

### Staphylokokkeninfektion nach Spritzenabzeß

### Status präsens:

Wegen Allergie wurde ein Patient mit den spezifischen Allergenen desensibilisiert. Nach einer dieser Injektionen entstand ein Spritzenabzeß im Gesäß. Trotz chirurgischer Intervention brach der Abzeß in das kleine Becken durch. Die Staphylokokken konnten aus dem Blut isoliert werden.
Da der Allgemeinzustand sich täglich verschlechterte wurde als ultimo ratio komplettes Adjuvans (Beispiel 2) eingesetzt.

### Vakzination:

Es wurden 1 ml komplettes Adjuvans mit einer inaktivierten St.aureus Keimdichte von 10⁹ Keimen pro ml i.m. appliziert.
Der kritische Zustand hielt noch 2 Tage an mit abnehmender Krise.

Nach 3 Tagen stand der Patient erstmalig wieder auf und nach weiteren 7 Tagen war er geheilt.

### Beispiel 8

### Akne vulgaris

### Status präsens:

Ein Patient seit etwa 10 Jahren mit Beginn der Pubertät hatte im Gesicht, am Rücken und auf der Brust vorwiegend in der Schweißrinne stark entstellende Aknefurunkel.
Die bakteriologische Untersuchung ergab Str.pyogenes und Corynebact acne mit guter antibiotischer Sensibilität. Da aber alle bisherigen Behandlungen einschließlich einer antibiotischen keine Besserung brachten, wurde versuchsweise vakziniert.

### Vakzination:

Es wurde komplettes Adjuvans (Beispiel 2) ohne zusätzliche Antigene appliziert.
Nach einer Woche begannen bereits die tieferen Aknefurunkel abzuheilen. Nach drei Wochen erfolgte eine zweite Vakzination mit der gleichen Dosis. Nach 6 Wochen von der ersten Immunisierung an gerechnet, entstand ein völlig neuer Gesichtsausdruck, da auch die Narben verblaßten und keine neuen Aknefurunkel entstanden.

### Beispiel 9

### Osteoporose

### Status präsens:

Eine Patientin mit Osteoporose, Ende des 4. Lebensjahrzehntes Menopause bereits eingetreten, war 4 Wochen in einer orthopädischen Klinik zur Stabilisierung des Bewegungsapparates zur Behandlung eingewiesen worden. Die Patientin konnte die Arbeiten im Haushalt nur mit Mühe oder fremder Hilfe verrichten. Da der Zustand auch nach dem Krankenhausaufenthalt sich nicht besserte, wurde der Versuch der Vakzination unternommen.

### Vakzination:

Im Abstand von 3 Wochen wurden jeweils 1 ml komplettes Adjuvans (Beispiel 2) ohne weitere Antigene i.m. appliziert.
14 Tagen nach der zweiten Injektion besserte sich das Befinden, so daß Arbeiten im Haushalt wieder leichter möglich wurden. Nach der dritten Vakzination ließen die Knochenschmerzen fast gänzlich nach und die Bewegungsfähigkeit wurde wieder völlig hergestellt.

Drei Monate nach Behandlungsbeginn begann der normale Menstruationszyklus ohne jegliche Komplikationen bei einem Jahr Nachbeobachtungszeit.

## Patentansprüche

1. Inkomplettes Adjuvans für Antigene, insbesondere wie Viren, Bakterien, Parasiten einschließlich ihrer Stoffwechselprodukte oder Teile der Strukturen von Viren, Bakterien und Parasiten zur Immunisierung, **dadurch gekennzeichnet**, daß das Adjuvans aus:
- 0,01% - 30 % Polydimethylsiloxane, die einen Polymerisationsgrad von n = 20 - 400 und eine kinematische Viskosität von 20 - 1000 mm²/s aufweisen,
- 0,01% - 15% Komplexemulgator mit einem HLB-Wert von 9-16,
- 45% - 99% biokompatible Salzlösung,
- 0,01 - 10 % Dimethylsulfoxid und
- 0, 0001 - 1 % Chelatbildner
besteht.

2. Inkomplettes Adjuvans nach den Ansprüchen 1 oder 2, **dadurch gekennzeichnet**, daß die Komplexemulgatoren aus aliphatischen Alkoholen der Kettenlängen C₁₀ bis C₁₀₀, aus Sorbitol- und/oder Glycerolfettsäureester und aus Polysorbaten bestehen.

3. Inkomplettes Adjuvans nach einem der Ansprüche 1 oder 2, **dadurch gekennzeichnet**, daß das Massenverhältnis von aliphatischen Alkoholen, Sorbitol- und/oder Glycerolfettsäureester und Polysorbaten 0,5-3 zu 0,2-4 zu 1-5 beträgt.

4. Inkomplettes Adjuvans nach einem der Ansprüche 1 bis 3, **dadurch gekennzeichnet**, daß als Chelatbildner Salze der Ethylendiamintetraessigsäure enthalten sind.

5. Inkomplettes Adjuvans nach einem der Ansprüche 1 bis 4, **dadurch gekennzeichnet**, daß zusätzlich mehrwertige, wasserlösliche Alkohole, bevorzugt Glycerol, in einer Konzentration von 0,01 - 10 % enthalten sind.

6. Komplettes Adjuvans für Antigene, insbesondere wie Viren, Bakterien, Parasiten einschließlich ihrer Stoffwechselprodukte oder Teile der Strukturen von Viren, Bakterien und Parasiten zur Immunisierung, **dadurch gekennzeichnet**, daß das Adjuvans aus inkomplettem Adjuvans gemäß einem der Ansprüche 1-5, Peptidoglycanen auf der Basis von speziesspezifischen St.aureus-Stämmen und/oder anderen Stämmen in einer Konzentration von 0,00001 bis 1 mg Eiweißstickstoff pro ml Adjuvans und wasserlöslichen natürlichen und/oder synthetischen Polymeren in einer Konzentration von 0,0001 bis 10 mg pro ml Adjuvans besteht.

7. Komplettes Adjuvans nach Anspruch 6, **dadurch gekennzeichnet**, daß als wasserlösliche natürliche und/oder synthetische Polymere
- Polyvinylpyrrolidon,
- Poly-6-Aminohexansäure,
- Polyvinylalkohol,
- Alkali- und Ammoniumalginate,
- Cellulose und partialsynthetische Celluloseester wie Methylcellulose, Ethylcellulose, Hydroxyethylcellulose, Ethylhydroxyethylcellulose, Natriumcarboxymethylcellulose
enthalten sind.

8. Verfahren zur Herstellung von kompletten Adjuvantien für Antigene gemäß einem der Ansprüche 6 oder 7, **dadurch gekennzeichnet**, daß zu einem inkompletten Adjuvans gemäß der Ansprüche 1 bis 5 eine Kochsalz enthaltende wäßrige Zusammensetzung von Peptidoglycanen auf der Basis von spezifischen St.aureus-Stämmen zugefügt wird, wobei zuvor zur Herstellung der Peptidoglycane speziesspezifische Stämme von St.aureus ausgewählt, auf Blutagar bebrütet, die geernteten Staphylokokken in gepufferter Phenolkochsalzlösung aufgenommen, inaktiviert, die inaktivierten Staphylokokken separiert, gewaschen, anschließend mit einer Kochsalz enthaltenden wäßrigen Lösung von wasserlöslichen natürlichen und/oder synthetischen Polymeren aufgenommen und im Autoklaven bei einer Temperatur von über 121°C behandelt werden.

9. Adjuvans gemäß der Ansprüche 1 oder 6 zur Verwendung für die Herstellung von Vakzinen und für die Immunisierung in der Human- und Veterinärmedizin.

## Claims

1. An incomplete adjuvant for antigens, such as viruses, bacteria and parasites, including their metabolic products or parts of the structures of viruses, bacteria and parasites for immunization, comprising:
- from 0,01% to 30% of polydimethylsiloxanes having a degree of polymerization of n from 20 to 400 and a kinematic viscosity of from 20 to 1000 mm²/s,
- from 0,01% to 15% of a complex emulsifier with an HLB of 9-16,
- from 45% to 99% of biocompatible salt solution,
- from 0,01% to 10% of dimethylsulfoxide and
- from 0,0001% to 1% of a chelating agent.

2. The incomplete adjuvant of claim 1 or 2, wherein said complex emulsifier consists of aliphatic alcohols with a chain length of C₁₀ to C₁₀₀, of fatty acid esters of sorbitol and/or glycerol and of polysorbates.

3. The incomplete adjuvant of any of claim 1 or 2, wherein the ratio by weight of said aliphatic alcohols to fatty acid esters of sorbitol and/or glycerol to polysorbates is 0.5-3 to 0.2-4 to 1-5.

4. The incomplete adjuvant of any of claim 1 to 3, wherein said chelating agent are salts of ethylenediaminetetraacetic acid.

5. The incomplete adjuvant of any of claim 1 to 4, further comprising multihydric, water-soluble alcohols, preferably glycerol, at a concentration of from 0,01% to 10%.

6. A complete adjuvant for antigens, such as viruses, bacteria and parasites, including their metabolic products or parts of the structures of viruses, bacteria and parasites for immunization, wherein the adjuvant consists of the incomplete adjuvant according to any of the claims 1 to 5, peptidoglycans based on species-specific strains of *Staphylococcus aureus* and/or other strains at a concentration of from 0.00001 to 1 mg of protein nitrogen per ml of adjuvant, water-soluble natural and/or synthetic polymers at a concentration of 0.0001 to 10 mg per ml of adjuvant.

7. The complete adjuvant of claim 6, wherein
- polyvinylpyrolidone
- poly-6-aminohexanoic acid
- polyvinyl alcohol
- alkali and ammonium alginate
- cellulose and partially synthetic cellulose esters, such as methylcellulose, ethylcellulose, hydroxyethylcellulose, ethylhydroxyethylcellulose, and sodiumcarboxymethylcellulose
are contained as water-soluble natural and/or synthetic polymers.

8. A method for preparing a complete adjuvant for antigens according to any of claim 6 or 7, which comprises adding a salt-containing aqueous composition of peptidoglycans based on a species-specific strain of *Staphylococcus aureus* to the incomplete adjuvant according to any of claim 1 to 5, previously, for the preparation of the peptidodyclans, species-specific strains of *Staphylococcus aureus* being selected, incubating said strains on blood agar, harvesting the incubated Staphylococci, taking up the harvested Staphylococci in a buffered phenol/salt solution, inactivating the taken up Staphylococci, separating and washing the inactivated Staphylococci, taking up said separated and washed Staphylococci in a salt-containg aqueous solution of a water-soluble, natural and/or synthetic polymer, and autoclaving the taken up Staphylococci at a temperature of more than 121°C.

9. The adjuvant of any of claim 1 or 6, comprising the use of said adjuvant for the preparation of vaccines and for immunization in human and veterinary medicine.

## Revendications

1. Adjuvant incomplet pour antigènes, tels que notamment virus, bactéries, parasites y compris leurs produits métaboliques, ou éléments de la structure de virus, bactéries et parasites, aux fins d'immunisation, **caractérisé en ce que** l'adjuvant consiste en :
- 0,01 % à 30 % de polydiméthylsiloxanes, présentant un degré de polymérisation de n = 20 à 400 et une viscosité cinématique de 20 à 1000 mm²/s,
- 0,01 % à 15 % d'émulsifiant complexe avec une valeur hlb de 9 à 16,
- 45 % à 99 % de solution saline biocompatible,
- 0,01 % à 10 % de diméthylsulfoxyde,
- 0,0001 à 1 % de chélateur.

2. Adjuvant incomplet selon les revendications 1 ou 2, **caractérisé en ce que** les émulsufiants complexes consistent en alcools aliphatiques de longueur de chaîne C10 à C100, en esters d'acides gras sorbitiques et/ou glycériques et en polysorbates.

3. Adjuvant incomplet selon l'une des revendications 1 ou 2, **caractérisé en ce que** le rapport des masses des alcools aliphatiques, des esters d'acides gras sorbitiques et/ou glycériques et des polysorbates est de 0,5-3 à 0,2-4 à 1-5.

4. Adjuvant incomplet selon l'une des revendications 1 à 3, **caractérisé en ce que** des sels d'acide acétique diaminetétraéthylénique sont compris comme chélateur.

5. Adjuvant incomplet selon l'une des revendications 1 à 4, **caractérisé en ce que** des alcools polyvalents solubles à l'eau, préférentiellement du glycérol, sont en outre compris dans une concentration de 0,01 % à 10 %.

6. Adjuvant complet pour antigènes, tels que notamment virus, bactéries, parasites y compris leurs produits métaboliques, ou éléments de la structure de virus, bactéries et parasites, aux fins d'immunisation, **caractérisé en ce que** l'adjuvant consiste en un adjuvant incomplet selon l'une des revendications 1 à 5, en peptidoglycanes sur la base de souches St. aureus d'espèces spécifiques et/ou d'autres souches, dans une concentration de 0,00001 à 1 mg d'azote protéique par ml d'adjuvant et de polymères naturels solubles dans l'eau et/ou synthétiques dans une concentration de 0,0001 à 10 mg par ml d'adjuvant.

7. Adjuvant complet selon revendication 6**, caractérisé en ce que** sont contenus comme polymères naturels solubles dans l'eau et/ou synthétiques :
- de la polyvinylpyrrolidone,
- de l'acide poly-hexa-amino-caproïque
- de l'alcool polyvinylique,
- des alginates d'alcali et d'ammonium,
- de la cellulose et des esters cellulosiques partiellement synthétiques tels que méthylcellulose, éthylcellulose, hydroxyéthylcellulose, éthylhydroxyéthylcellulose, carboxyméthylcellulose de sodium.

8. Procédé pour la production d'adjuvants complets pour antigènes selon l'une des revendications 6 ou 7, **caractérisé en ce qu**' est ajoutée une composition aqueuse et contenant du chlorure de sodium de peptidoglycanes sur la base de souches St. aureus spécifiques, des souches St. aureus d'espèces spécifiques étant au préalable sélectionnées pour la production des peptidoglycanes, incubées sur agar sanguin, les staphylocoques produits étant recueillis dans un sérum phénolique tamponné et inactivés, lesdits staphylocoques inactivés étant séparés, lavés, et absorbés ensuite avec une solution aqueuse contenant du chlorure de sodium par des polymères naturels solubles dans l'eau et/ou synthétiques, et traités en autoclave à une température supérieure à 121 ° C.

9. Adjuvant selon les revendications 1 ou 6, utilisable pour la réalisation de vaccins et aux fins d'immunisation en médecine humaine et vétérinaire.
